# EUROPEAN PATENT SPECIFICATION

(11) **EP 3 810 285 B1**
(45) Date of publication and mention of the grant of the patent: **12.02.2025**
(21) Application number: 18924932.9
(22) Date of filing: 21.12.2018
(51) Int. Cl.: A61K 31/4245, A61P 35/02, C07C 215/28, C07D 271/06

(54) **SPHINGOSINE PATHWAY MODULATING COMPOUNDS FOR THE TREATMENT OF CANCERS**
VERBINDUNGEN ZUR MODULIERUNG DES SPHINGOSIN-SIGNALWEGS FÜR DIE BEHANDLUNG VON KREBS
COMPOSÉS MODULANT LA VOIE DE LA SPHINGOSINE POUR LE TRAITEMENT DE CANCERS

(30) Priority: 25.06.2018 US 201816017303
(43) Date of publication of application: 28.04.2021
(73) Proprietor: Enzo Biochem, Inc., Farmingdale, NY 11735 (US); Elazar, Rabbani, New York, NY 10003 (US); Donegan, James J., Long Beach, NY 11561 (US); Diamond, Paul, New York, NY 10025 (US)
(72) Inventor: RABBANI, Elazar, New York, NY 10003 (US); DONEGAN, James J., NY 11561 (US); DIAMOND, Paul, New York, NY 10025 (US)
(74) Representative: Vossius & Partner Patentanwälte Rechtsanwälte mbB
(86) International application number: PCT/US2018/067131
(87) International publication number: WO 2020/005313

(56) References cited:
- WO-A1-2018/035292
- WO-A1-2018/093591
- WO-A1-2018/093591
- WO-A2-2018/237379
- US-A1- 2009 318 389
- US-A1- 2010 035 959
- US-A1- 2010 035 959
- SCOTT ET AL.: "Ozanimod (RPC1063) is a potent sphingosine-1-phosphate receptor-1 (S1P1) and receptor-5 (S1P5) agonist with autoimmune disease-modifying activity", BRITISH JOURNAL OF PHARMACOLOGY, vol. 173, no. 11, 15 March 2016 (2016-03-15), pages 1778 - 1792, XP055422423, DOI: 10.1111/bph.13476

## Description

### FIELD OF THE INVENTION

The invention as defined in the claims relates to the field of pharmaceutical treatment of pancreatic cancer.

### BACKGROUND

Sphingosine-1-phosphate (S1P) was discovered to be a bioactive signaling molecule over 20 years ago. Studies have since identified two related kinases, sphingosine kinase 1 and 2 (a/k/a sphingosine kinase "type I" and "type II" respectively, and SphK1 and SphK2 respectively), which catalyze the phosphorylation of sphingosine to S1P. Extracellular S1P can bind to and activate each of five S1P-specific, G protein-coupled receptors (designated S1PR₁₋₅) to regulate cellular and physiological processes in an autocrine or paracrine manner. Selective inhibitors of each of sphingosine kinase 1 and 2, as well as both non-selective and selective agonists of S1PRs, have been developed and are known in the art. WO 2018093591 A1 describes a therapeutic agent in combination with ozanimod (RPC-1063) for use in treating leukemia or lymphoma.

### SUMMARY

One embodiment of the invention provides ozanimod or a pharmaceutically acceptable salt thereof for use in the treatment of pancreatic cancer in a mammalian subject, such as a human. Another embodiment of the invention provides ozanimod or a pharmaceutically acceptable salt thereof in combination with SK1-I or a pharmaceutically acceptable salt thereof for use in the treatment of pancreatic cancer in a mammalian subject, such as a human.

A related embodiment of the invention provides a pharmaceutical composition comprising a therapeutically effective amount of ozanimod or a pharmaceutically acceptable salt thereof for use in the treatment of pancreatic cancer in a mammalian subject, such as a human patient.

Another embodiment of the invention provides a pharmaceutical composition comprising a therapeutically effective amount of ozanimod or a pharmaceutically acceptable salt thereof for use in the treatment of pancreatic cancer in a mammalian subject, such as a human, further comprising a therapeutically effective amount of SK1-I or a pharmaceutically acceptable salt thereof.

Ozanimod (RPC1063) is a sphingosine-1-phosphate receptor agonist of the sphingosine-1-phosphate receptor-1 (S1P₁) and the sphingosine-1-phosphate receptor-5 (S1P₅).

A sphingosine kinase type I inhibitor, such as SK1-I, is disclosed in U.S. Patent No. 8,372,888 and/or 8,314,151.

Immune checkpoint inhibitors, which may be monoclonal antibodies, can be one or more selected from the group consisting of: PD-1 inhibitors such as mAbs Pembrolizumab (Keytruda^{®}) and Nivolumab (Opdivo^{®}); PD-L1 inhibitors such as mAbs Atezolizumab (Tecentriq^{®}), Avelumab (Bavencio^{®}), and Durvalumab (Imfinzi^{®}); and CTLA-4 inhibitors such as mAb Ipilimumab (Yervoy^{®}), and V-domain Ig Suppressor of T Cell Activation (VISTA) inhibitors such as mAb JNJ-61610588 (ImmuNext Inc.).

### BRIEF DESCRIPTION OF THE DRAWINGS

FIG. 1 shows MTT assay data (72 hours) for various concentrations of ozanimod for four human hepatocellular carcinoma cell lines.
FIG. 2 shows MTT assay data (72 hours) for various concentrations of ABC294640 for four human hepatocellular carcinoma cell lines.
FIG. 3 shows MTT assay data (72 hours) for various concentrations of SK1-I for four human hepatocellular carcinoma cell lines.
FIG. 4A shows MTT assay data (48 hours) for various concentrations of SK1-I for three human hepatocellular carcinoma cell lines. FIG. 4B shows MTT assay data (48 hours) for various concentrations of PF-543, a super potent SphK1 inhibitor, for the same three human hepatocellular carcinoma cell lines shown in FIG. 4A.
FIGS. 5A-D show apoptosis assay data for various concentrations of SK1-I and no-drug control for Huh7 cells. SK1-I strongly induced apoptosis in the Huh7 cells.
FIGS. 6A-D show apoptosis assay data for various concentrations of SK1-I and no-drug control for PLC-PRF5 cells. SK1-I strongly induced apoptosis in the PLC-PRF5 cells.
FIGS. 7A-D show apoptosis assay data for various concentrations of SK1-I and no-drug control for Hep 3B cells. SK1-I strongly induced apoptosis in the Hep 3B cells.
FIG. 8A-D show apoptosis assay data for various concentrations of SK1-I and no-drug control for Hep G2 cells. SK1-I strongly induced apoptosis in the Hep G2 cells.
FIGS. 9A-D show apoptosis assay data for various concentrations of ozanimod and no-drug control for Huh7 cells. Ozanimod strongly induced apoptosis in the Huh7 cells.
FIGS. 10A-D show apoptosis assay data for various concentrations of ozanimod and no-drug control for PLC-PRF5 cells. Ozanimod strongly induced apoptosis in the PLC-PRF5 cells.
FIGS. 11A-D show apoptosis assay data for various concentrations of ozanimod and no-drug control for Hep 3B cells. Ozanimod strongly induced apoptosis in the Hep 3B cells.
FIGS. 12A-D show apoptosis assay data for various concentrations of ozanimod and no-drug control for Hep G2 cells. Ozanimod strongly induced apoptosis in the Hep G2 cells.
FIGS. 13A-D shows apoptosis assay data for various concentrations of ABC294640 and no-drug control for Huh7 cells. ABC294640 failed to induce apoptosis in the Huh7 cells.
FIGS. 14A-D show apoptosis assay data for various concentrations of ABC294640 and no-drug control for PLC-PRF5 cells. ABC294640 did not substantially induce apoptosis in the PLC-PRF5 cells.
FIGS. 15A-D show apoptosis assay data for various concentrations of ABC294640 and no-drug control for Hep 3B cells. ABC294640 did not substantially induce apoptosis in the Hep 3B cells.
FIGS. 16A-D show apoptosis assay data for various concentrations of ABC294640 and no-drug control for Hep G2 cells. ABC294640 failed to induce apoptosis in the Hep G2 cells.
FIGS. 17A-C show apoptosis assay data for various concentrations of SK1-I and no-drug control for Jurkat cells (human T-cell leukemia cell line). SK1-I strongly induced apoptosis in the Jurkat cells.
FIGS. 18A-C show apoptosis assay data for various concentrations of ozanimod and no-drug control for Jurkat cells. Ozanimod strongly induced apoptosis in the Jurkat cells.
FIGS. 19A-D show apoptosis assay data for various concentrations of SK1-I and no-drug control for primary human hepatocytes. SK1-I did not induce apoptosis of the primary human hepatocytes at any concentration tested.
FIGS. 20A-D show apoptosis assay data for various concentrations of ozanimod and no-drug control for primary human hepatocytes. Ozanimod did not induce apoptosis of the primary human hepatocytes at any concentration tested.
FIG. 21 shows the cell viability effects of 5 µM SK1-I alone, 5 µM ozanimod alone, and the combination of 5 µM SK1-I and 5 µM ozanimod on Jurkat cells, Jurkat cells cultured with IL-2, PBMCs, and PBMCs cultured with IL-2.
FIG. 22A shows the cell viability effects of different concentrations of SK1-I and ozanimod, each alone and in combination, on pancreatic cell line PanC-1 cells. FIG. 22B shows the cell viability effects of different concentrations of SK1-I and ozanimod, each alone and in combination, on pancreatic cancer cell line BxPC-3 cells.

### DETAILED DESCRIPTION

The scope of the present invention is defined in the claims; any other disclosure in the present description not protected by the appended claims, regardless of whether it is indicated as being preferred or exemplified, is provided for information purposes, only.

The invention provides as defined in the claims, new therapeutic uses of sphingosine kinase-1 inhibitor SK1-I and selective sphingosine-1-phosphate receptor agonist ozanimod for treating pancreatic cancer.

Sphingosine kinase 1 inhibitors described herein may include any of those disclosed in U.S. Patent Nos. 8,372,888 and/or 8,314,151or pharmaceutically acceptable salts thereof. The sphingosine kinase I inhibitor used in accordance with the present invention as defined is the claims is (E,2R,3S)-2-(methylamino)-5-(4-pentylphenyl)pent-4-ene-1,3-diol (also known as SK1-I), or a pharmaceutically acceptable salt thereof such as a hydrochloride salt. The structure of SK1-I is shown below.

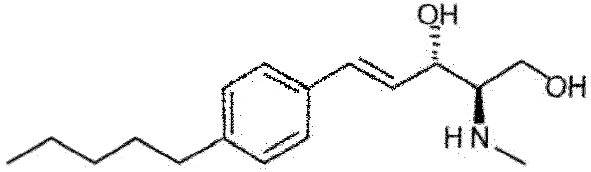

*See also* Paugh et al., Blood, 2008 112: 1382-1391.

A sphingosine kinase I inhibitor described may be a compound having the structure 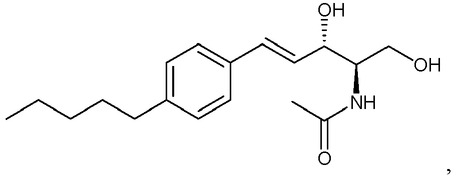 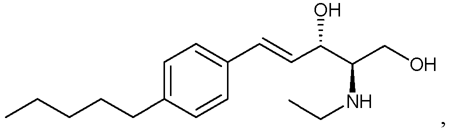 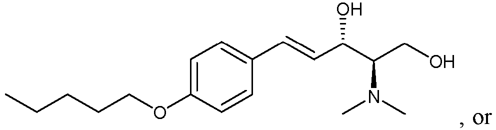 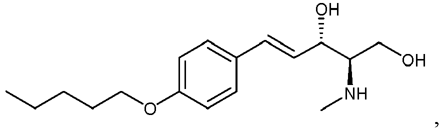 or a pharmaceutically acceptable salt of the compound such as a hydrochloride salt.

A sphingosine kinase I inhibitor described may be a compound having the structure 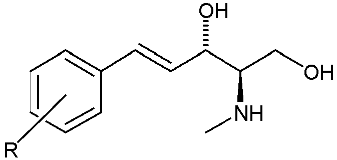
wherein R is selected from a straight carbon chain, a branched carbon chain, a straight carbon chain comprising one or more heteroatoms, a branched carbon chain comprising one or more heteroatoms, a cyclic ring, a heterocyclic ring, an aromatic ring, a hetero-aromatic ring, or any combination of the foregoing,
or a pharmaceutically acceptable salt thereof such as
a hydrochloride salt.

A sphingosine kinase I inhibitor described may be a compound having the structure 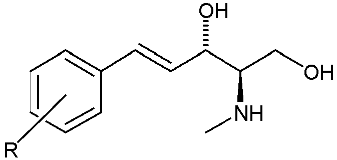
wherein R is 3,4-dimethoxy, 4-phenyl or 3-pentyl,
or a pharmaceutically acceptable salt thereof such as
a hydrochloride salt.

Sphingosine-1-phosphate receptor agonists described may be any of those disclosed in any of U.S. Pub. Nos. 20110172202, 20130231326, and 20150299149, or pharmaceutically acceptable salts thereof. The agonists may be agonists of one or both of sphingosine-1-phosphate receptor-1 (S1P₁) and sphingosine-1-phosphate receptor-5 (S1P₅) and may have little or at least no substantial agonist activity against other sphingosine-1-phosphate receptors (in a mammal such as a human). The sphingosine-1-phosphate receptor agonist used in accordance with the present invention as defined in the claims is 5-[3-[(1S)-1-(2-hydroxyethylamino)-2,3-dihydro-1H-inden-4-yl]-1,2,4-oxadiazol-5-yl]-2-propan-2-yloxybenzonitrile (also known as ozanimod and RPC1063) or a pharmaceutically acceptable salt thereof such as a hydrochloride salt. The structure of ozanimod is shown below.

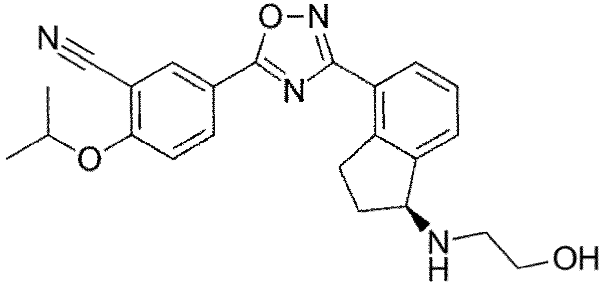

*See also* Scott et al., British Journal of Pharmacology 2016 173:1778-1792.

A sphingosine-1-phosphate receptor agonist described may be etrasimod or a pharmaceutically acceptable salt thereof such as a hydrochloride salt. The structure of etrasimod is shown below.

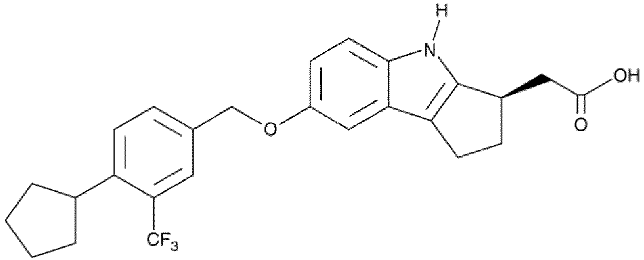

A sphingosine-1-phosphate receptor agonist described may be amiselimod or a pharmaceutically acceptable salt thereof such as a hydrochloride salt. The structure of amiselimod is shown below.

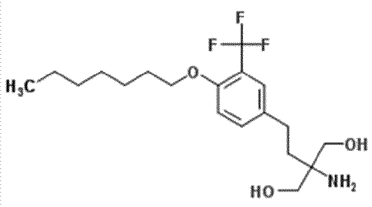

ABC294640 (also known as Yeliva^{®}) used in the experiments presented herein is a reported sphingosine kinase-2 selective inhibitor, namely the compound (7S)-3-(4-chlorophenyl)-N-(pyridin-4-ylmethyl)adamantane-1-carboxamide. The structure of ABC294640 is shown below.

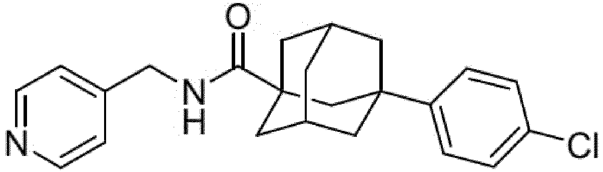

*See also* French et al., J. Pharmacol. Exp. Ther. 2010, 333, 129-139.

### Experiments

Experiments which do not fall under the subject-matter of the appended claims do not form part of the present invention and aid for reference purposes.

MTT cell viability assays evaluating the effect of different concentrations of each of ozanimod, ABC294640 and SK1-I on four human hepatocellular carcinoma cell lines, Hep G2, Hep 3B, Huh 7 and PLC-PRF5 were performed. These four cell lines were selected for the study because they are among HCC cell lines whose gene expression profiles most closely resemble those of primary HCC tumors. *See* Chen et al., BMC Medical Genomics 2015, 8(Suppl 2):S5. The following concentrations of the compounds were tested. Ozanimod: 200 µM, 111.1 µM, 61.73 µM, 34.29 µM, 19.05 µM, 10.58 µM, and 0 µM. ABC294640: 200 µM, 111.1 µM, 61.73 µM, 34.29 µM, 19.05 µM, 10.58 µM, and 0 µM. SK1-I: 20 µM, 11.11 µM, 6.173 µM, 3.429 µM, 1.905 µM, 1.058 µM, and 0 µM.

The following MTT assay protocol was followed.
- Prepared stock solutions: 50 mM ozanimod, 50 mM ABC294640, 10 mM SK1-I in DMSO.
- Plated 20000 cells in 160 µl medium per well in 96-well plates for each cell line and incubated at 37°C overnight.
- Prepared compound serial dilutions: for ozanimod and ABC294640: dilute stock 50 mM 1:50 in medium to 1000 µM; for SK1-I, dilute stock 10 mM 1:50 in medium to 100 µM, them make serial 1:1.8 fold serial dilution to the titration. For negative control, diluted DMSO 1:50 to medium.
- Added 40 µl negative control and serially titrated compounds to 160 µl of cells for each cell line. Performed in triplicate for each cell line.
- Incubated at 37°C for 72 hours.
- Performed assay using Vybrant MTT Cell Proliferation Assay kit (V-13154) (Molecular Probes) from Thermo Fisher Scientific (Waltham, MA USA).

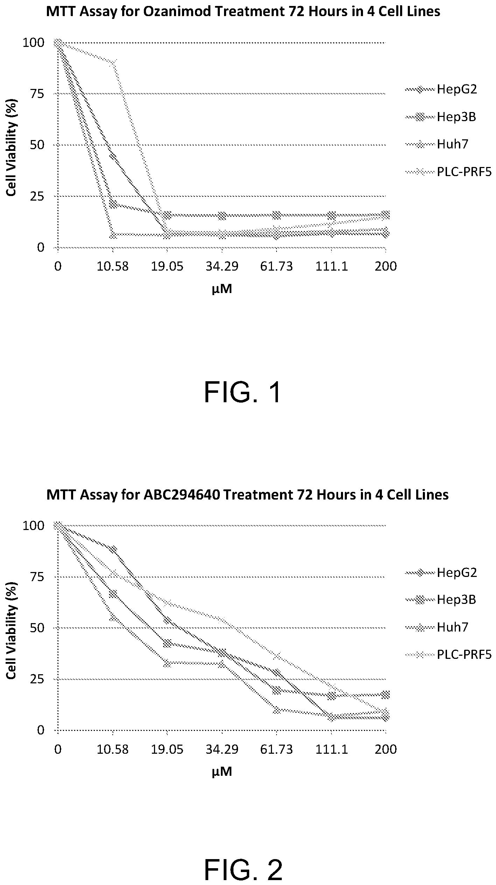

The results of these 72-hour treatment MTT assays are shown in FIGS. 1-3 as follows.

FIG. 1 shows the MTT assay data (72 hours) for ozanimod for the four human hepatocellular carcinoma cell lines.

FIG. 2 shows the MTT assay data (72 hours) for ABC294640 for the four human hepatocellular carcinoma cell lines.

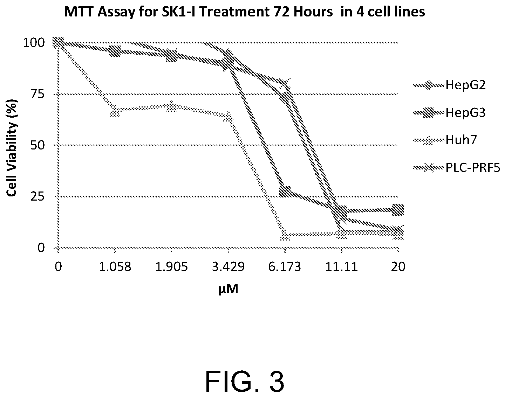

FIG. 3 shows the MTT assay data (72 hours) for SK1-I for the four human hepatocellular carcinoma cell lines.

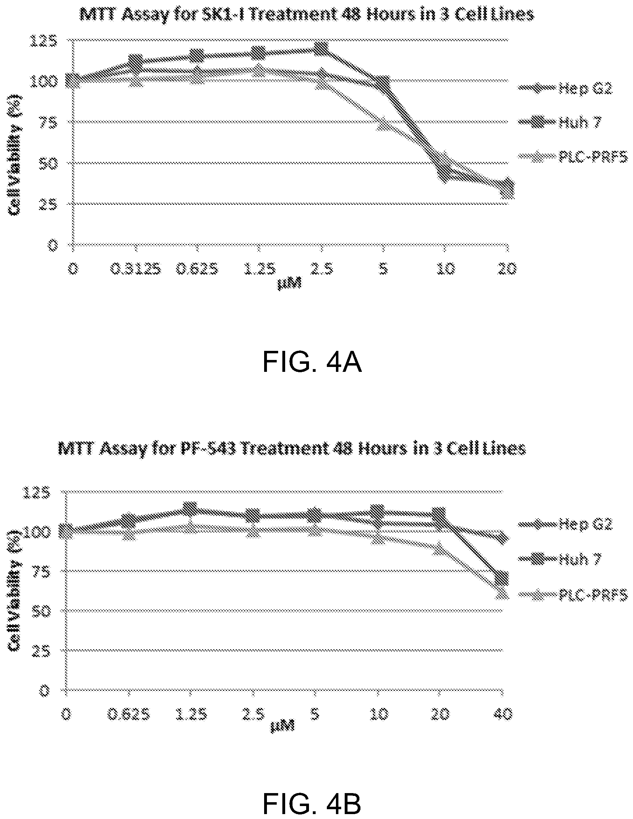

48-hour treatment MTT assays were also performed as follows. FIG. 4A shows MTT assay data (48 hours) for various concentrations of SK1-I for three human hepatocellular carcinoma cell lines, Hep G2, Huh 7, and PLC-PRF5. FIG. 4B shows MTT assay data (48 hours) for various concentrations of PF-543, a super potent SphK1 inhibitor (*see* Schnute et al., Biochem. J. (2012) 444, 79-88), for the same three human hepatocellular carcinoma cell lines shown in FIG. 4A. This data shows that SK1-I is more effective at killing hepatocellular carcinoma cells than PF-543 despite the latter drug's much greater potency in inhibiting SphK1.

Apoptosis/necrosis assays evaluating the effect of different concentrations of each of ozanimod, ABC294640 and SK1-I on the four human hepatocellular carcinoma cell lines, Hep G2, Hep 3B, Huh 7 and PLC-PRF5 were also performed.

The protocol used for the apoptosis assays was:
- Plated cells in 6-well plates and incubated at 37°C overnight.
- Prepared concentrations of test compound and control compound DMSO in media.
   Ozanimod: 5 µM, 10 µM, and 20 µM.
   ABC294640: 10 µM, 20 µM, and 40 µM.
   SK1-I: 10 µM, 20 µM, and 40 µM.
   Control: 0.2% DMSO.
- Aspirated the medium from the 6-well plates and added test compound concentrations in media or 0.2% DMSO control in media. Incubated at 37°C for 24 hours.
- Collected and processed the cells following the flow cytometry protocol of the GFP Certified^{®} Apoptosis/Necrosis detection kit from Enzo Life Sciences, Inc. (product no. ENZ-51002; Farmingdale, NY, USA).

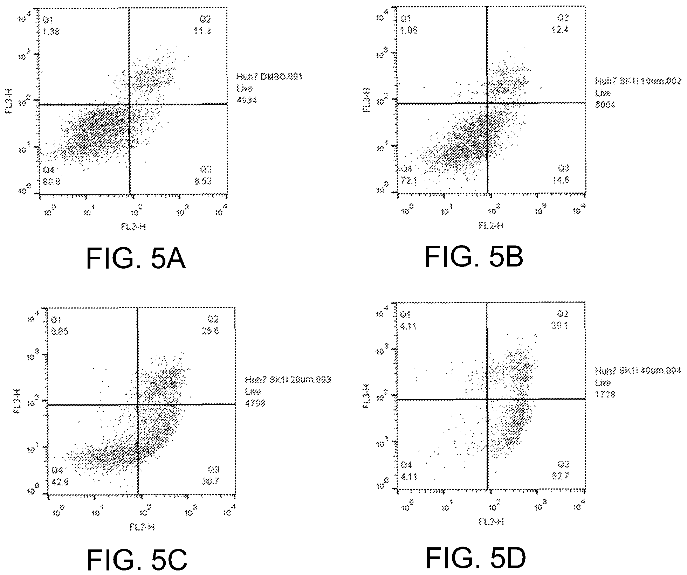

FIGS. 5-16 present graphs plotting the data from these 24-hour apoptosis assays for the different concentrations of compounds and no-drug control for the various cell lines. Channel FL2 picks up the apoptosis signal and channel FL3 picks up the necrosis signal. Data points in Quadrant 3 (Q3) in the graphs corresponds to cells undergoing apoptosis (cells positive for the apoptosis detection reagent of the assay). Data points in Quadrant 2 (Q2) in the graphs corresponds to cells that are positive for the apoptosis detection reagent and positive for the necrosis detection reagent of the assay (indicative of late-stage apoptosis).

FIGS. 5A-D show the apoptosis assay data for various concentrations of SK1-I and no-drug control for Huh7 cells. FIG. 5A shows the results for no-drug control. FIG. 5B shows the results for treatment with 10 µM SK1-I. FIG. 5C shows the results for treatment with 20 µM SK1-I. FIG. 5D shows the results for treatment with 40 µM SK1-I. SK1-I strongly induced apoptosis in the Huh7 cells.

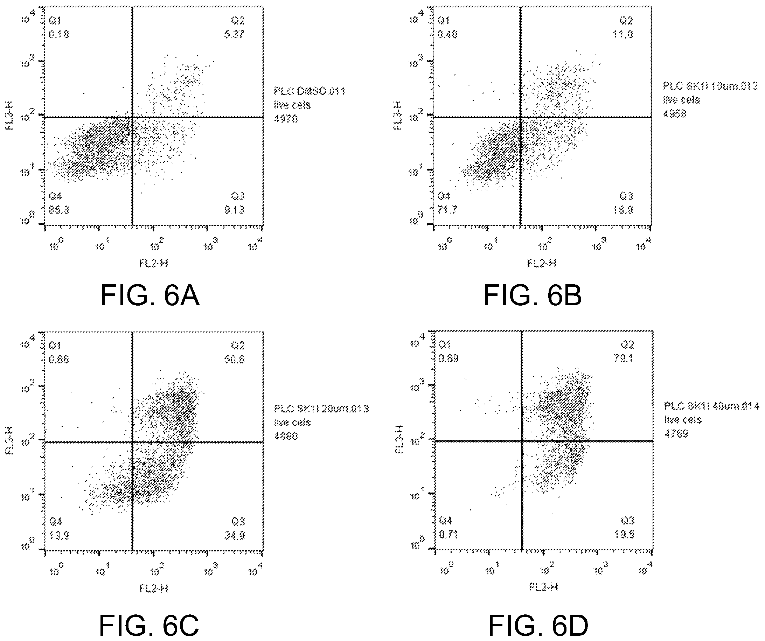

FIGS. 6A-D show the apoptosis assay data for various concentrations of SK1-I and no-drug control for PLC-PRF5 cells. FIG. 6A shows the results for no-drug control. FIG. 6B shows the results for treatment with 10 µM SK1-I. FIG. 6C shows the results for treatment with 20 µM SK1-I. FIG. 6D shows the results for treatment with 40 µM SK1-I. SK1-I strongly induced apoptosis in the PLC-PRF5 cells.

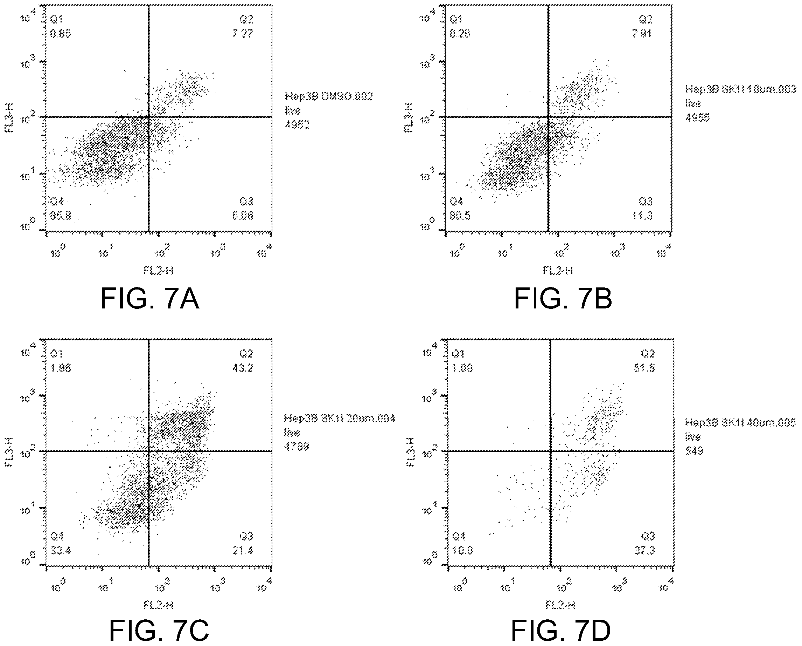

FIGS. 7A-D show the apoptosis assay data for various concentrations of SK1-I and no-drug control for Hep 3B cells. FIG. 7A shows the results for no-drug control. FIG. 7B shows the results for treatment with 10 µM SK1-I. FIG. 7C shows the results for treatment with 20 µM SK1-I. FIG. 7D shows the results for treatment with 40 µM SK1-I. SK1-I strongly induced apoptosis in the Hep 3B cells.

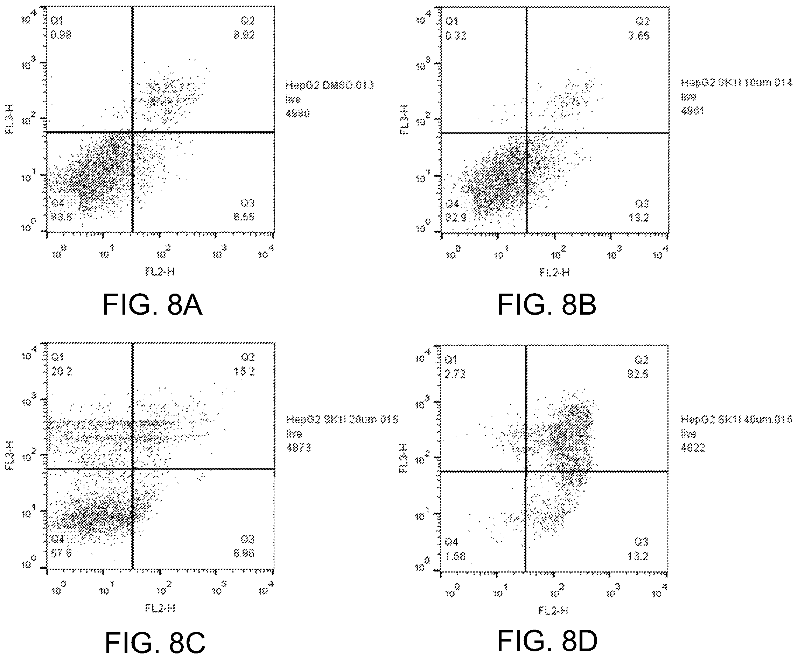

FIGS. 8A-D show the apoptosis assay data for various concentrations of SK1-I and no-drug control for Hep G2 cells. FIG. 8A shows the results for no-drug control. FIG. 8B shows the results for treatment with 10 µM SK1-I. FIG. 8C shows the results for treatment with 20 µM SK1-I. FIG. 8D shows the results for treatment with 40 µM SK1-I. SK1-I strongly induced apoptosis in the Hep G2 cells.

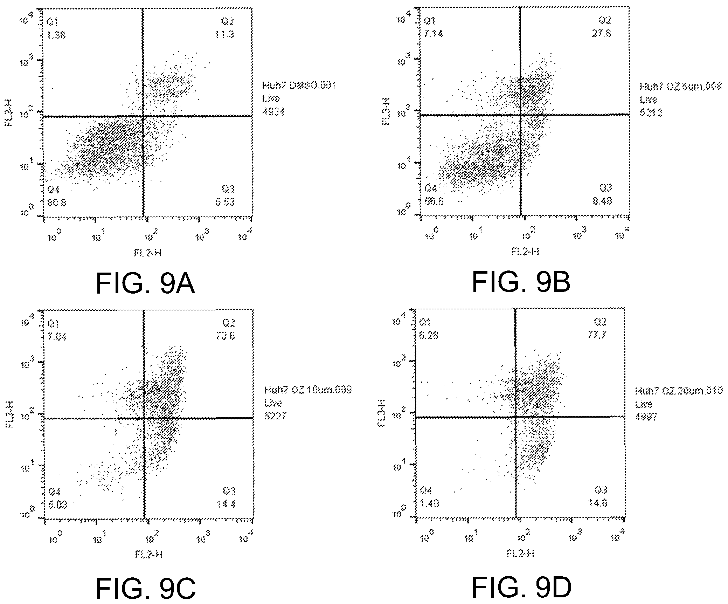

FIGS. 9A-D show the apoptosis assay data for various concentrations of ozanimod and no-drug control for Huh7 cells. FIG. 9A shows the results for no-drug control. FIG. 9B shows the results for treatment with 5 µM ozanimod. FIG. 9C shows the results for treatment with 10 µM ozanimod. FIG. 9D shows the results for treatment with 20 µM ozanimod. Ozanimod strongly induced apoptosis in the Huh7 cells.

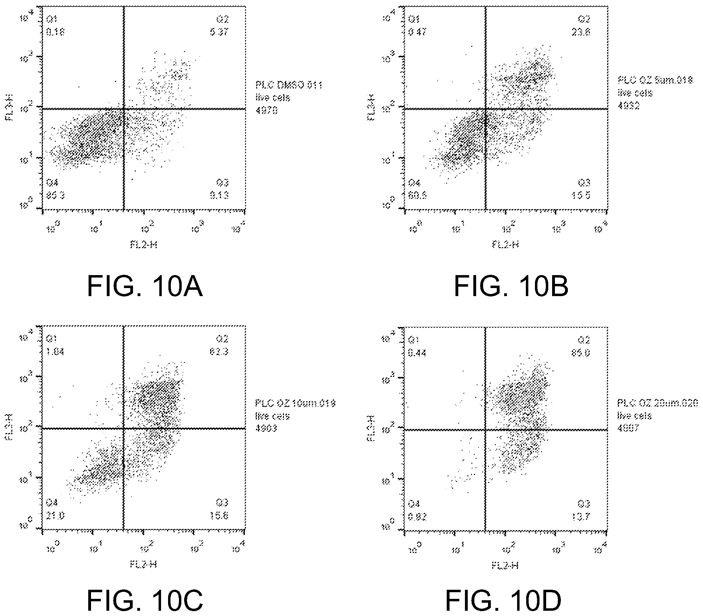

FIGS. 10A-D show the apoptosis assay data for various concentrations of ozanimod and no-drug control for PLC-PRF5 cells. FIG. 10A shows the results for no-drug control. FIG. 10B shows the results for treatment with 5 µM ozanimod. FIG. 10C shows the results for treatment with 10 µM ozanimod. FIG. 10D shows the results for treatment with 20 µM ozanimod. Ozanimod strongly induced apoptosis in the PLC-PRF5 cells.

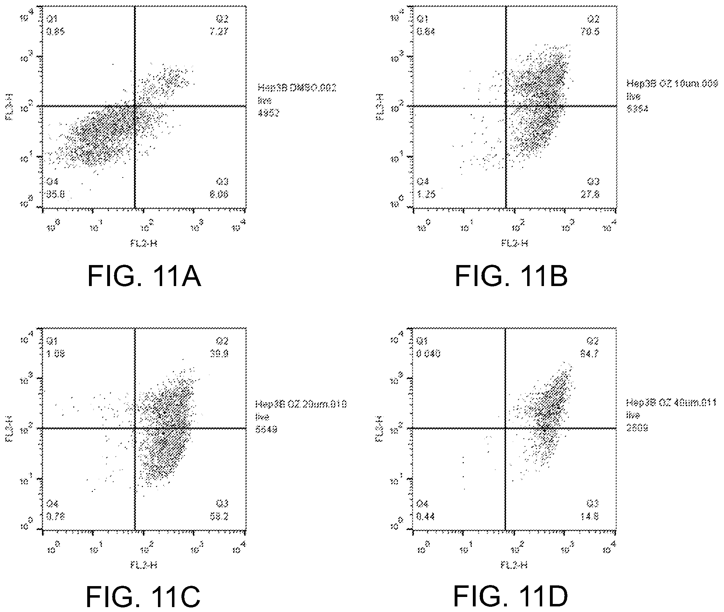

FIGS. 11A-D show the apoptosis assay data for various concentrations of ozanimod and no-drug control for Hep 3B cells. FIG. 11A shows the results for no-drug control. FIG. 11B shows the results for treatment with 10 µM ozanimod. FIG. 11C shows the results for treatment with 20 µM ozanimod. FIG. 11D shows the results for treatment with 40 µM ozanimod. Ozanimod strongly induced apoptosis in the Hep 3B cells.

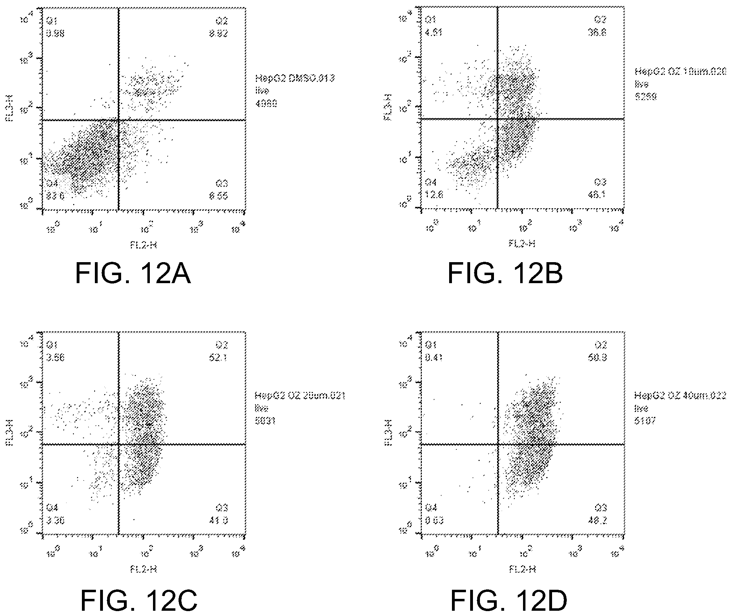

FIGS. 12A-D show the apoptosis assay data for various concentrations of ozanimod and no-drug control for Hep G2 cells. FIG. 12A shows the results for no-drug control. FIG. 12B shows the results for treatment with 10 µM ozanimod. FIG. 12C shows the results for treatment with 20 µM ozanimod. FIG. 12D shows the results for treatment with 40 µM ozanimod. Ozanimod strongly induced apoptosis in the Hep G2 cells.

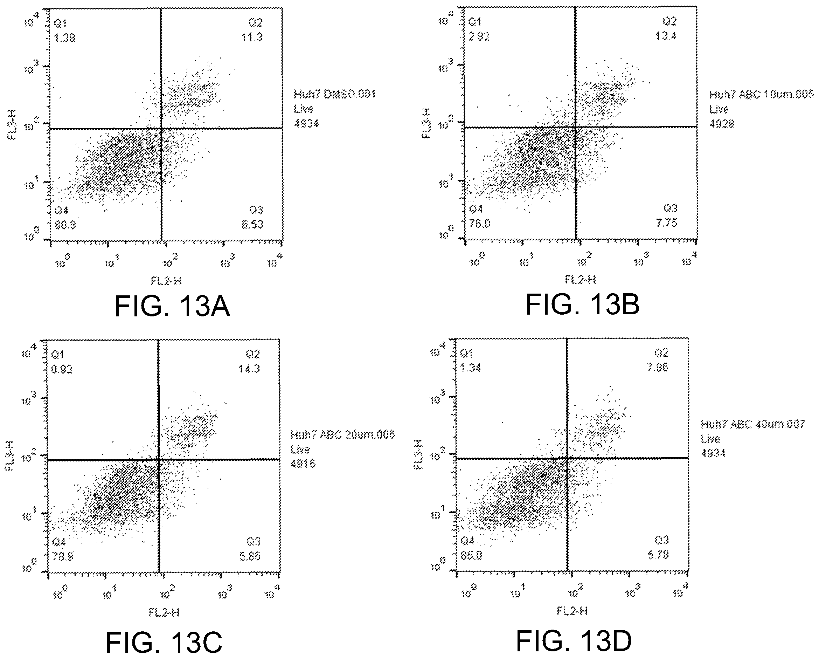

FIGS. 13A-D show the apoptosis assay data for various concentrations of ABC294640 and no-drug control for Huh7 cells. FIG. 13A shows the results for no-drug control. FIG. 13B shows the results for treatment with 10 µM ABC294640. FIG. 13C shows the results for treatment with 20 µM ABC294640. FIG. 13D shows the results for treatment with 40 µM ABC294640. ABC294640 failed to induce apoptosis in the Huh7 cells.

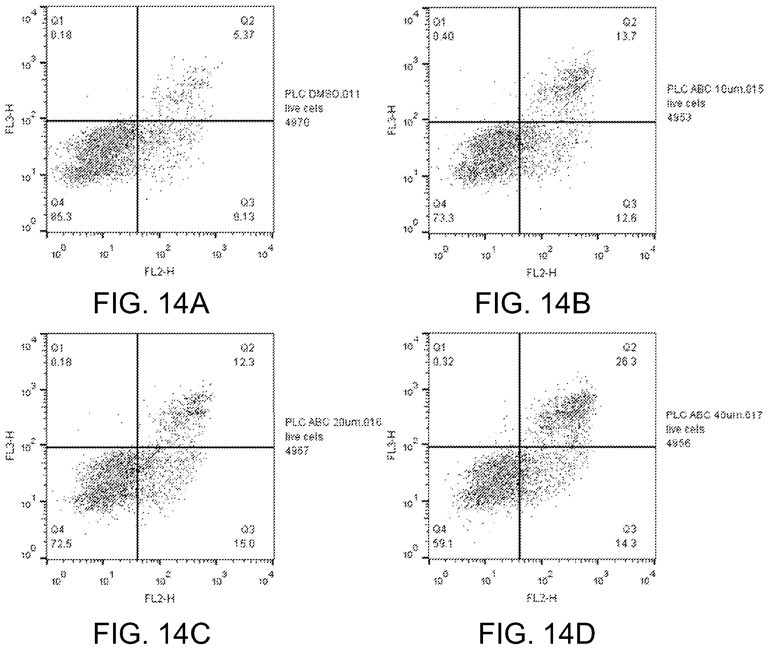

FIGS. 14A-D show the apoptosis assay data for various concentrations of ABC294640 and no-drug control for PLC-PRF5 cells. FIG. 14A shows the results for no-drug control. FIG. 14B shows the results for treatment with 10 µM ABC294640. FIG. 14C shows the results for treatment with 20 µM ABC294640. FIG. 14D shows the results for treatment with 40 µM ABC294640. ABC294640 did not substantially induce apoptosis in the PLC-PRF5 cells.

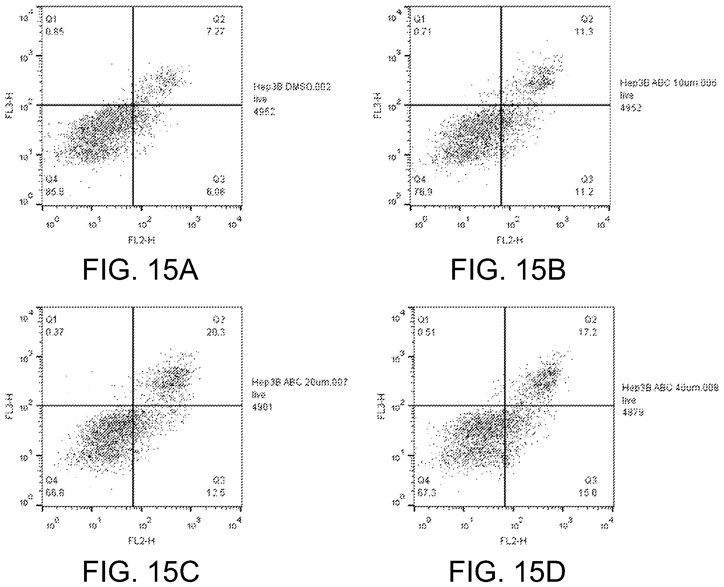

FIGS. 15A-D show the apoptosis assay data for various concentrations of ABC294640 and no-drug control for Hep 3B cells. FIG. 15A shows the results for no-drug control. FIG. 15B shows the results for treatment with 10 µM ABC294640. FIG. 15C shows the results for treatment with 20 µM ABC294640. FIG. 15D shows the results for treatment with 40 µM ABC294640. ABC294640 did not substantially induce apoptosis in the Hep 3B cells.

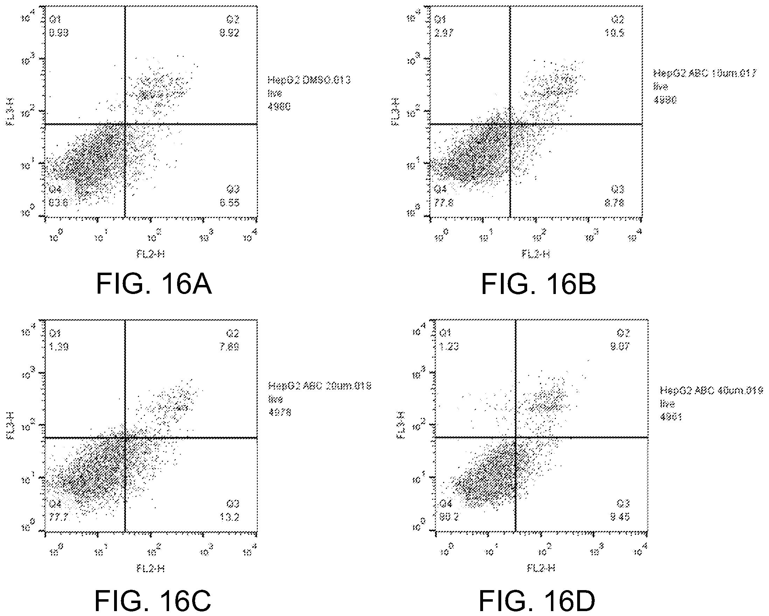

FIGS. 16A-D show the apoptosis assay data for various concentrations of ABC294640 and no-drug control for Hep G2 cells. FIG. 16A shows the results for no-drug control. FIG. 16B shows the results for treatment with 10 µM ABC294640. FIG. 16C shows the results for treatment with 20 µM ABC294640. FIG. 16D shows the results for treatment with 40 µM ABC294640. ABC294640 failed to induce apoptosis in the Hep G2 cells.

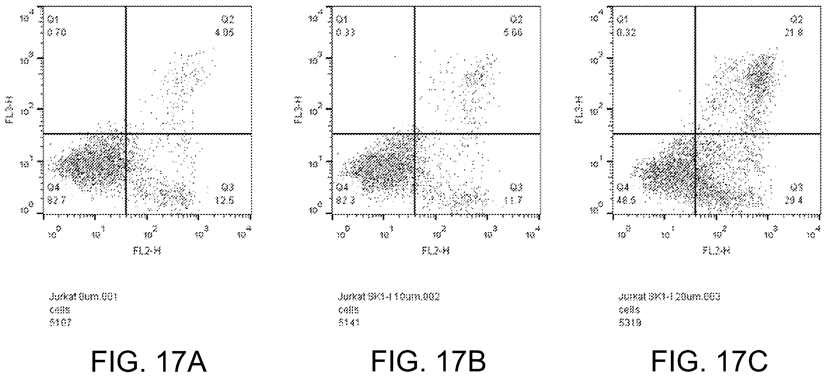

FIGS. 17A-C show apoptosis assay data for various concentrations of SK1-I and no-drug control for Jurkat cells (human T-cell leukemia cell line). FIG. 17A shows the results for no-drug control. FIG. 17B shows the results for treatment with 10 µM SK1-I. FIG. 17C shows the results for treatment with 20 µM SK1-I. SK1-I strongly induced apoptosis in the Jurkat cells.

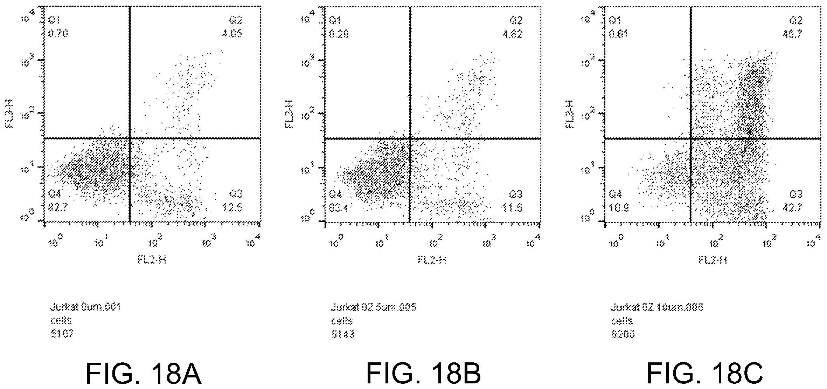

FIGS. 18A-C show apoptosis assay data for various concentrations of ozanimod and no-drug control for Jurkat cells. FIG. 18A shows the results for no-drug control. FIG. 18B shows the results for treatment with 5 µM ozanimod. FIG. 18C shows the results for treatment with 10 µM ozanimod. Ozanimod strongly induced apoptosis in the Jurkat cells.

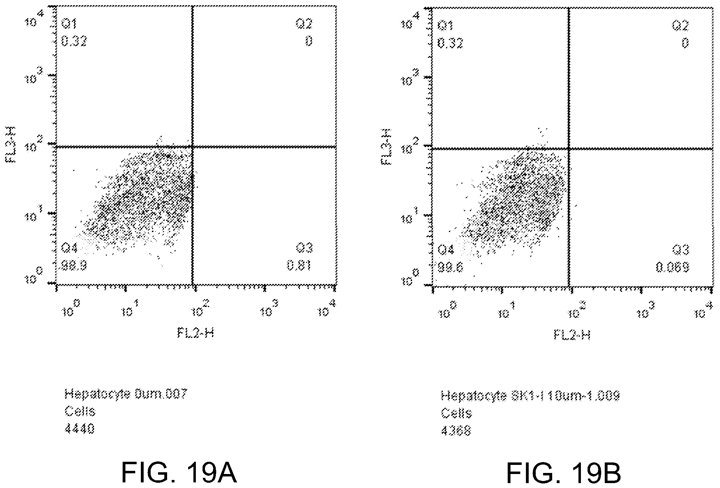

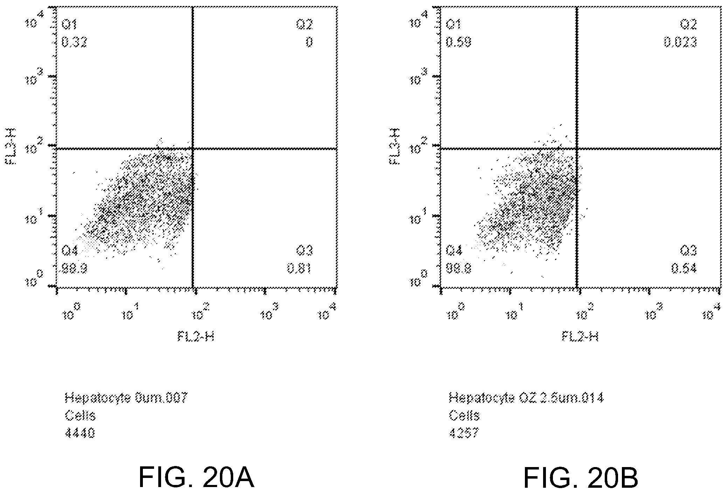

In still further experiments, the effects of SK1-I and ozanimod on normal (non-cancerous) primary human hepatocytes were investigated. Fresh human hepatocytes in a 12-well plate (HUF12) were obtained from Triangle Research Labs (Durham, NC, USA; part of Lonza Group) and handled according to the supplier's protocol. The shipping medium was aspirated from each well and replaced with 1 ml per well of warm Hepatocyte Maintenance Medium. The plate was then placed in a 5% CO₂ incubator at 37°C and the hepatocytes were allowed to acclimate overnight. The Hepatocyte Maintenance Medium was replaced before treatment with drug or no-drug control, and the hepatocytes were treated with 0 µM (no-drug control), 10 µM, 20 µM, or 40 µM SK1-I or 0 µM, 2.5 µM, 5 µM, or 10 µM ozanimod for 24 hours under incubation. The cells were then harvested and analyzed using the GFP Certified^{®} Apoptosis/Necrosis detection kit. The results are shown in FIGS. 19A-D for SK1-I and FIGS. 20A-D for ozanimod as follows.

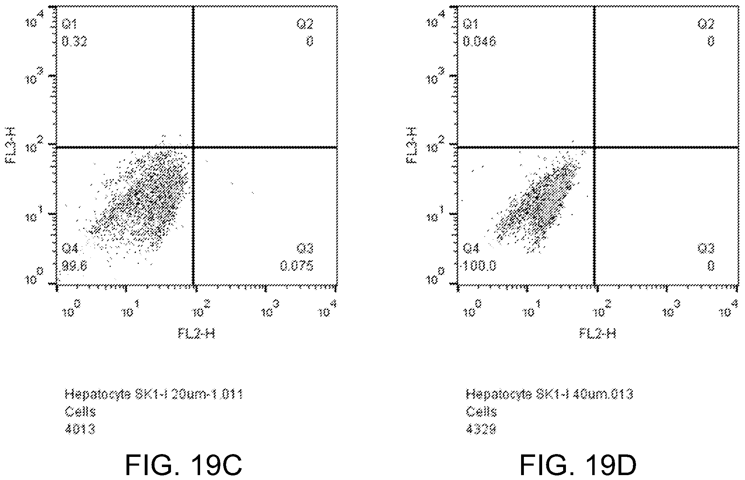

FIGS. 19A-D show apoptosis assay data for various concentrations of SK1-I and no-drug control for primary human hepatocytes. FIG. 19A shows the results for no-drug control. FIG. 19B shows the results for treatment with 10 µM SK1-I. FIG. 19C shows the results for treatment with 20 µM SK1-I. FIG. 19D shows the results for treatment with 40 µM SK1-I. SK1-I did not induce apoptosis of the primary human hepatocytes at any concentration tested.

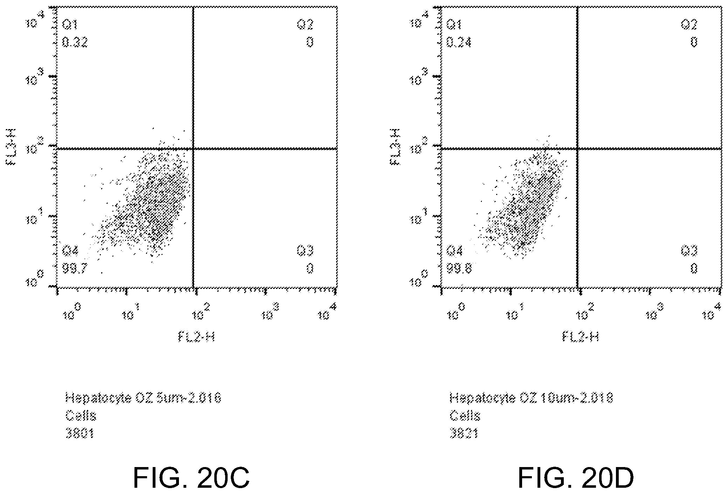

FIGS. 20A-D show apoptosis assay data for various concentrations of ozanimod and no-drug control for primary human hepatocytes. FIG. 20A shows the results for no-drug control. FIG. 20B shows the results for treatment with 2.5 µM ozanimod. FIG. 20C shows the results for treatment with 5 µM ozanimod. FIG. 20D shows the results for treatment with 10 µM ozanimod. Ozanimod did not induce apoptosis of the primary human hepatocytes at any concentration tested.

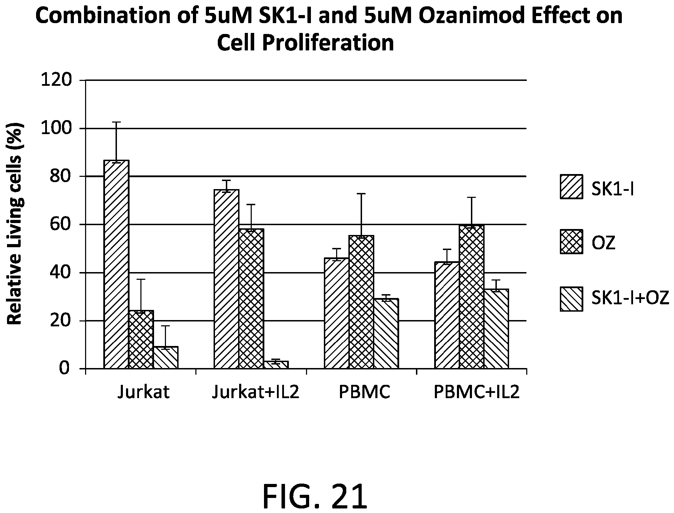

FIG. 21 shows the cell viability effects of 5 µM SK1-I alone, 5 µM ozanimod alone, and the combination of 5 µM SK1-I and 5 µM ozanimod on Jurkat cells, Jurkat cells cultured with IL-2, PBMCs, and PBMCs cultured with IL-2. The data shows, for example, that ozanimod strongly decreases viability of Jurkat cells without IL-2 and the combination of SK1-I and ozanimod even more dramatically decreases viability of Jurkat cells both without and with IL-2, while the combination of agents was not nearly as detrimental to peripheral blood mononuclear cells (PBMC) irrespective of IL-2.

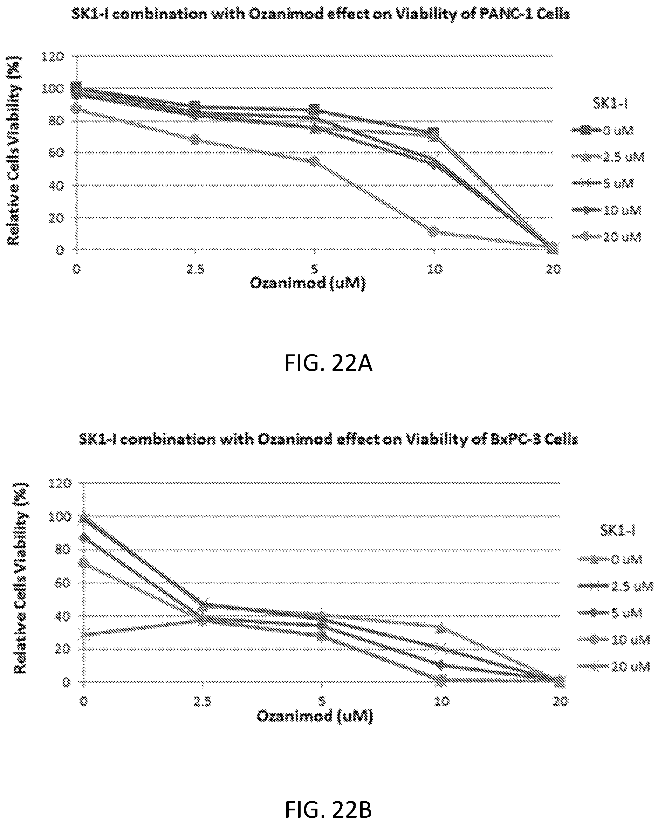

FIG. 22A shows the cell viability effects of different concentrations of SK1-I and ozanimod, each alone and in combination, on pancreatic cell line PanC-1 cells.

FIG. 22B shows the cell viability effects of different concentrations of SK1-I and ozanimod, each alone and in combination, on pancreatic cancer cell line BxPC-3 cells.

Described and not falling within claimed subject-matter is that the therapeutic use may further include one or more immune checkpoint inhibitors which may be monoclonal antibodies (mABs). The immune checkpoint inhibitor may be selected from the group consisting of the following: PD-1 inhibitors such as mAbs Pembrolizumab (Keytruda^{®}) and Nivolumab (Opdivo^{®}); PD-L1 inhibitors such as mAbs Atezolizumab (Tecentriq^{®}), Avelumab (Bavencio^{®}), and Durvalumab (Imfinzi^{®}); and CTLA-4 inhibitors such as mAb Ipilimumab (Yervoy^{®}); and V-domain Ig Suppressor of T Cell Activation (VISTA) inhibitors such as mAb JNJ-61610588 (ImmuNext Inc.) is/are to be co-administered.

Immune checkpoint inhibitors may, for example, be administered by injection in the dosages described herein and/or at the currently approved dosages for said inhibitors.

The amount of compound that is effective for use in the treatment or prevention of a condition, alone or in combination with other compounds, may be determined by standard techniques. In addition, *in vitro* and/or *in vivo* assays may optionally be employed to help identify optimal dosage ranges. The precise dose to be employed will also depend on, *e.g.,* the route of administration and the seriousness of the condition and can be decided according to the judgment of a practitioner and/or each patient's circumstances. In other examples thereof, variations will necessarily occur depending upon the weight and physical condition (*e.g.,* hepatic and renal function) of the patient being treated, the affliction to be treated, the severity of the symptoms, the frequency of the dosage interval, the presence of any deleterious side-effects, and the particular compound utilized, among other things.

Administration may be as a single dose or as a divided dose. In one aspect, an effective dosage is to be administered once per month until the condition is abated. In another aspect, the effective dosage is to be administered once per week, or twice per week or three times per week until the condition is abated. An effective dosage may, for example, be administered at least once daily or at least or at least once every two-days, or at least once every three days, four days, five days, six days or seven days. In another aspect, an effective dosage amount is to be administered about every 24 h until the condition is abated. In another aspect, an effective dosage amount is to be administered about every 12 h until the condition is abated. In another aspect, an effective dosage amount is to be administered about every 8 h until the condition is abated. In another aspect an effective dosage amount is to be administered about every 6 h until the condition is abated. In another aspect, an effective dosage amount is to be administered about every 4 h until the condition is abated.

The therapeutically effective doses/amounts of the pharmaceutical compounds disclosed herein may be expressed in terms of the amount of the compound(s) or pharmaceutically acceptable salts thereof administered per unit body weight of the subject per day of treatment, or the total amount administered per day of treatment. A daily dose may, for example, be at least 0.005 mg/kg of body weight, at least 0.01 mg/kg of body weight, at least 0.025 mg/kg of body weight, at least 0.05 mg/kg of body weight, at least 0.1 mg/kg of body weight, at least 0.2 mg/kg of body weight, at least 0.3 mg/kg of body weight, at least 0.4 mg/kg of body weight, at least 0.5 mg/kg of body weight, at least 0.6 mg/kg of body weight, at least 0.7 mg/kg of body weight, at least 0.8 mg/kg of body weight, at least 0.9 mg/kg of body weight, at least 1 mg/kg of body weight, at least 1.5 mg/kg of body weight, at least 2 mg/kg of body weight, at least 2.5 mg/kg of body weight, at least 3 mg/kg of body weight, at least 3.5 mg/kg of body weight, at least 4 mg/kg of body weight, at least 4.5 mg/kg of body weight, at least 5 mg/kg of body weight, or at one of said doses. A total daily dose may, for example, be in the range of 0.005 mg/kg to 5 mg/kg or any subrange or value therein, such as 0.025 to 5 mg/kg body weight, such as 0.05 to 5 mg/kg body weight. A total daily dose may, for example be in the range of 0.1 mg to 1,000 mg total or any subrange or value therein, such as 0.1 mg to 1,000 mg, such as 0.1 mg to 100 mg, such as 0.1 mg to 50 mg, such as 0.5 mg to 50 mg, such as 1.0 mg to 50 mg, such as 5 mg to 50 mg, or 0.1 mg to 10 mg, such as 0.5 mg to 10 mg. For SK1-I and related SphK1 inhibitors disclosed U.S. Patent Nos. 8,372,888 and 8,314,151, and pharmaceutically acceptable salts thereof, a daily dose for human subjects may, for example, also be in the range of 0.5 mg/kg to 5 mg/kg or any subrange or value therein, such as 1 mg/kg to 4 mg/kg, such as 1 mg/kg to 3 mg/kg, or, for example, a total daily dose of 5 mg to 50 mg or any subrange or value therein, such as 10 mg to 40 mg, such as 20 mg to 40 mg. For ozanimod, its active metabolites and related sphingosine-1-phosphate receptor agonists disclosed in U.S. Pub Nos. 20110172202, 20130231326, and 20150299149, and pharmaceutically acceptable salts thereof, a daily dose for human subjects may, for example, also be in the range of 1 mg to 50 mg or any subrange or value therein, or 0.1 mg to 10 mg or any subrange or value therein, such as 0.1 mg to 5 mg, such as 0.5 to 5 mg, such as 0.5 mg to 2.5 mg, such as 0.5 mg to 1.5 mg. A pharmaceutical composition may, for example, include a daily dose amount of the compound as set forth herein.

The duration of treatment by administration of a therapeutic compound or combination may continue for a plurality of days, such as for at least one week, at least two weeks, at least three weeks, at least four weeks, at least two months, at least three months, at least four months, at least five months, at least six months, at least seven months, at least eight months, at least nine months, at least 10 months, at least 11 months, at least 12 months, at least 1 ½ years, at least 2 years, at least three years, at least four years, or may continue indefinitely.

The terms co-administration and co-administering mean that each of the things being co-administered is to be administered to a subject in such temporal proximity that each (or its active metabolite(s)) is present in active form in the subject for an at least partially overlapping period of time. Accordingly, co-administration may include, simultaneous administration, such as when the things being administered are part of the same pharmaceutical composition, or sequential administration of the things being co-administered, for example, within the same day of each other, within 12 hours of each other, within 6 hours of each other, within 3 hours of each other, within 1 hours of each other, or within 15 minutes of each other. The things being administered may be administered by the same route, such as by oral ingestion or injection, or by different routes.

Pharmaceutically acceptable salts and the selection and preparation thereof are well known in the art. Such salts include hydrochloride, citrate, glycolate, fumarate, malate, tartrate, mesylate, esylate, cinnamate, isethionate, sulfate, phosphate, diphosphate, nitrate, hydrobromide, hydroiodide, succinate, formate, acetate, dichloroacetate, lactate, p-toluenesulfonate, pamitate, pidolate, pamoate, salicylate, 4-aminosalicylate, benzoate, 4-acetamido benzoate, glutamate, aspartate, glycolate, adipate, alginate, ascorbate, besylate, camphorate, camphorsulfonate, camsylate, caprate, caproate, cyclamate, laurylsulfate, edisylate, gentisate, galactarate, gluceptate, gluconate, glucuronate, oxoglutarate, hippurate, lactobionate, malonate, maleate, mandalate, napsylate, napadisylate, oxalate, oleate, sebacate, stearate, succinate, thiocyanate, undecylenate, and xinafoate.

It should be noted that the indefinite articles "a" and "an" and the definite article "the" are used in the present application to mean one or more unless the context clearly dictates otherwise. Further, the term "or" is used in the present application to mean the disjunctive "or" or the conjunctive "and." It should also be understood that wherever in the present application the term comprising or including (or a term of similar scope) is recited in connection with the description of any embodiment or part thereof, a corresponding embodiment or part thereof reciting instead the term consisting essentially of or the term consisting of (or a term of similar scope) is also disclosed. It should also be understood that wherever a chemical structure or chemical group disclosed herein has one or more stereoisomers or stereoisomeric forms, corresponding embodiments directed to each of the stereoisomers or stereoisomeric forms individually or to any combination of the particular stereoisomers or stereoisomeric forms are also intended to be disclosed.

## Claims

1. Ozanimod or a pharmaceutically acceptable salt thereof for use in the treatment of pancreatic cancer in a mammalian subject.

2. Hydrochloride salt of ozanimod for use according to claim 1.

3. Ozanimod or a pharmaceutically acceptable salt thereof in combination with SK1-I or a pharmaceutically acceptable salt thereof for use according to claim 1.

4. Hydrochloride salt of ozanimod in combination with the hydrochloride salt of SK1-I for use according to claim 1.

5. A pharmaceutical composition comprising a therapeutically effective amount of ozanimod or a pharmaceutically acceptable salt thereof for use in the treatment of pancreatic cancer in a mammalian subject.

6. The pharmaceutical composition for use according to claim 5, further comprising a therapeutically effective amount of SK1-I or a pharmaceutically acceptable salt thereof.

7. The pharmaceutical composition for use according to claim 5, or 6, further comprising at least one pharmaceutically acceptable excipient.

8. Compound or composition for use according to any one of claims 1 to 4, wherein the mammalian subject is a human.

## Patentansprüche

1. Ozanimod oder ein pharmazeutisch verträgliches Salz davon zur Verwendung bei der Behandlung von Pankreaskrebs in einem Säugerindividuum.

2. Hydrochloridsalz von Ozanimod zur Verwendung nach Anspruch 1.

3. Ozanimod oder ein pharmazeutisch verträgliches Salz davon in Kombination mit SK1-I oder einem pharmazeutisch verträglichen Salz davon zur Verwendung nach Anspruch 1.

4. Hydrochloridsalz von Ozanimod in Kombination mit dem Hydrochloridsalz von SK1-I zur Verwendung nach Anspruch 1.

5. Pharmazeutische Zusammensetzung, umfassend eine therapeutisch wirksame Menge von Ozanimod oder ein pharmazeutisch verträgliches Salz davon zur Verwendung bei der Behandlung von Pankreaskrebs in einem Säugerindividuum.

6. Pharmazeutische Zusammensetzung zur Verwendung nach Anspruch 5, des Weiteren umfassend eine therapeutisch wirksame Menge von SK1-I oder ein pharmazeutisch verträgliches Salz davon.

7. Pharmazeutische Zusammensetzung zur Verwendung nach Anspruch 5 oder 6, des Weiteren umfassend mindestens einen pharmazeutisch verträglichen Exzipienten.

8. Verbindung oder Zusammensetzung zur Verwendung nach einem der Ansprüche 1 bis 4, wobei das Säugerindividuum ein Mensch ist.

## Revendications

1. Ozanimod ou un sel pharmaceutiquement acceptable de celui-ci pour utilisation dans le traitement du cancer du pancréas chez un sujet mammifère.

2. Sel de chlorhydrate d'ozanimod pour utilisation selon la revendication 1.

3. Ozanimod ou un sel pharmaceutiquement acceptable de celui-ci en combinaison avec le SK1-I ou un sel pharmaceutiquement acceptable de celui-ci pour utilisation selon la revendication 1.

4. Sel de chlorhydrate d'ozanimod en combinaison avec le sel de chlorhydrate de SK1-I pour utilisation selon la revendication 1.

5. Composition pharmaceutique comprenant une quantité thérapeutiquement efficace d'ozanimod ou d'un sel pharmaceutiquement acceptable de celui-ci pour utilisation dans le traitement du cancer du pancréas chez un sujet mammifère.

6. Composition pharmaceutique pour utilisation selon la revendication 5, comprenant en outre une quantité thérapeutiquement efficace de SK1-I ou d'un sel pharmaceutiquement acceptable de celui-ci.

7. Composition pharmaceutique pour utilisation selon la revendication 5, ou 6, comprenant en outre au moins un excipient pharmaceutiquement acceptable.

8. Composé ou composition pour utilisation selon l'une quelconque des revendications 1 à 4, le sujet mammifère étant un être humain.
